# EUROPEAN PATENT SPECIFICATION

(11) **EP 3 565 636 B1**
(45) Date of publication and mention of the grant of the patent: **03.01.2024**
(21) Application number: 17889852.4
(22) Date of filing: 22.11.2017
(51) Int. Cl.: A61P 25/00, A61P 25/14, A61P 25/16, A61P 25/28

(54) **USE OF GUT MICROBIOTA IN THE DIAGNOSIS OF PARKINSON'S DISEASE**
VERWENDUNG DER DARMFLORA BEI DER DIAGNOSE VON MORBUS PARKINSON
UTILISATION DU MICROBIOTE INTESTINAL DANS LE DIAGNOSTIC DE LA MALADIE DE PARKINSON

(30) Priority: 09.01.2017 US 201762444081 P
(43) Date of publication of application: 13.11.2019
(73) Proprietor: California Institute of Technology, Pasadena, CA 91125 (US)
(72) Inventor: MAZMANIAN, Sarkis, K., Glendale, CA 91206 (US); SAMPSON, Timothy, R., Pasadena, CA 91125 (US)
(74) Representative: Zacco Denmark A/S
(86) International application number: PCT/US2017/063108
(87) International publication number: WO 2018/128722

(56) References cited:
- WO-A2-2010/056985
- US-A1- 2006 167 057
- US-A1- 2013 115 257
- OTZEN D ET AL: "We find them here, we find them there: Functional bacterial amyloid", CELLULAR AND MOLECULAR LIFE SCIENCES, BIRKHÄUSER-VERLAG, BA, vol. 65, no. 6, 24 November 2007 (2007-11-24), pages 910-927, XP019583895, ISSN: 1420-9071
- KELLY SCHWARTZ ET AL: "Microbial amyloids - functions and interactions within the host", CURRENT OPINION IN MICROBIOLOGY, vol. 16, no. 1, 1 February 2013 (2013-02-01), pages 93-99, XP055697136, GB ISSN: 1369-5274, DOI: 10.1016/j.mib.2012.12.001
- SHU G. CHEN ET AL: "Exposure to the Functional Bacterial Amyloid Protein Curli Enhances Alpha-Synuclein Aggregation in Aged Fischer 344 Rats and Caenorhabditis elegans", SCIENTIFIC REPORTS, vol. 6, no. 1, 6 October 2016 (2016-10-06) , XP055497738, DOI: 10.1038/srep34477
- SAMPSON TIMOTHY R ET AL: "Gut Microbiota Regulate Motor Deficits and Neuroinflammation in a Model of Parkinson's Disease", CELL, ELSEVIER, AMSTERDAM, NL, vol. 167, no. 6, 1 December 2016 (2016-12-01), page 1469, XP029830881, ISSN: 0092-8674, DOI: 10.1016/J.CELL.2016.11.018

## Description

The present application claims priority under 35 U.S.C. § 119(e) to U.S. Provisional Application No. 62/444,081 filed on January 9, 2017.

### REFERENCE TO ELECTRONIC SEQUENCE LISTING

The present application is being filed along with a sequence listing in electronic format. The sequence listing is provided as a file entitled CALTE120WO.txt, created and last saved November 21, 2017 which is 12,157 Bytes in size.

### BACKGROUND

### Field

The present invention relates to a method of determining if a subject has a risk of developing parkinsonism.

### Description of the Related Art

Neurological dysfunction is the basis of numerous human diseases. Behavioral, psychiatric, and neurodegenerative disorders often display hallmark neuropathologies within the central nervous system (CNS). One neuropathology, amyloidosis, results from aberrant aggregation of specific neuronal proteins that disrupt many cellular functions. Affected tissues often contain insoluble aggregates of proteins that display altered conformations, a feature believed to contribute to an estimated 50 distinct human diseases (Sacchettini and Kelly, Nat Rev Drug Discov 1:267-275(2002)).

Neurodegenerative amyloid disorders, including Alzheimer's, Huntington's, and Parkinson's diseases (PD), are associated with various distinct amyloid proteins (Brettschneider et al., Nat Rev Neurosci 16:109-120 (2015)). PD is the second most common neurodegenerative disease in the United States, affecting an estimated 1 million people and 1% of the US population over 60 years of age (Nalls et al., 2014). Worldwide, about 3 million patients and caregivers suffer from the often-debilitating symptoms of PD, which involve motor deficits including tremors, muscle rigidity, bradykinesia, and impaired gait. It is a multifactorial disorder that has a strong environmental component, as less than 10% of cases are hereditary (Nalls et al., 2014). Aggregation of α-synuclein (αSyn) is thought to be pathogenic in a family of diseases termed synucleinopathies, which includes PD, multiple system atrophy, and Lewy body disease (Brettschneider et al., 2015; Luk et al., Science 338:949-953 (2012); Prusiner et al., Proc Natl Acad Sci USA 112:E5308-5317 (2015)). αSyn aggregation is a stepwise process, leading to oligomeric species and intransient fibrils that accumulate within neurons. Dopaminergic neurons of the substantia nigra pars compacta (SNpc) appear particularly vulnerable to effects of αSyn aggregates. Dopamine modulators are a first-line therapeutic in PD; however, treatments can carry serious side effects and often lose effectiveness (Jenner, Nat Rev Neurosci 9:665-677 (2008)). Discovery of safe and effective therapeutics and diagnoses are needed to address the increasing burden of PD in an ever-aging population, a paradoxical consequence of mankind's achievements in increased lifespan.

### SUMMARY

The invention is defined in the appended claims.

In some embodiments, the present disclosure provides methods of treating parkinsonism in a subject, comprising administering a composition comprising an amyloid inhibitor to the subject. In some embodiments, the present disclosure provides a method of delaying or reducing the likelihood of onset of parkinsonism in a subject, comprising administering a composition comprising an amyloid inhibitor to a subject at risk of developing parkinsonism. In some embodiments, the present disclosure provides methods for improving a motor deficit in a subject suffering from parkinsonism, comprising administering a composition comprising an amyloid inhibitor to the subject. These foregoing embodiments are not part of the invention.

In some embodiments, for any of the foregoing methods, the amyloid inhibitor is an inhibitor of a bacterial amyloid protein. In some embodiments, for any of the foregoing methods, the amyloid inhibitor inhibits the interaction of a bacterial amyloid protein or a subunit thereof with synuclein. In some embodiments, for any one of the foregoing methods, the amyloid inhibitor inhibits the seeding or nucleation of synuclein aggregation by a bacterial amyloid protein or a subunit thereof. In some embodiments, for any of the foregoing methods, the bacterial amyloid protein comprises a curli subunit. In some embodiments, for any of the foregoing methods, the parkinsonism is a primary or idiopathic parkinsonism, secondary or acquired parkinsonism, hereditary parkinsonism, Parkinson's disease, Parkinson's disease plus syndromes or multiple system degeneration, or any combination thereof. In some embodiments, for any of the foregoing methods, the parkinsonism is Parkinson's disease.

In some embodiments, the present disclosure provides methods of diagnosing parkinsonism in a subject, comprising determining the presence and/or level of one or more amyloid-producing bacterial species in the subject, in which the presence and/or abnormal level of the one or more amyloid-producing bacterial species indicates that the subject has a risk of developing, or is suffering from a symptom of parkinsonism. In some embodiments, for the foregoing methods, the parkinsonism is Parkinson's disease. In some embodiments, for any of the foregoing methods, the amyloid-producing bacterial species is an amyloid-producing *E. coli.* In some embodiments, for any of the foregoing methods, the presence and/or level of the one or more amyloid-producing bacterial species is determined by an analytical method as described herein, for example direct culture, antibody binding, ELISA, western blot, lateral flow assay, no-wash assay, sequencing, PCR, RT-PCR, qPCR or two or more of any of these, for example, two, three, four, or five of the listed items. In some embodiments, for any of the foregoing methods, determining the presence and/or level of the one or more amyloid-producing bacterial species in the subject comprises detecting a presence and/or level of the amyloid in a sample of the subject, for example a stool, urine, gut biopsy, cerebral spinal fluid (CSF), CNS biopsy, peripheral nervous tissue biopsy, urinary or urogenital tissue biopsy, tissue derived from the vagus nerve, an *in vitro* culture of any of the listed sample types, or a combination of two or more of any of the listed items. In some embodiments, for any of the foregoing methods, determining the presence and/or level of the one or more amyloid-producing bacterial species in the subject comprises detecting a presence and/or level of an aggregate of the amyloid in a sample of the subject, for example a stool, urine, gut biopsy, cerebral spinal fluid (CSF), CNS biopsy, peripheral nervous tissue biopsy, urinary or urogenital tissue biopsy, tissue derived from the vagus nerve, an *in vitro* culture of any of the listed items, or a combination of two or more of any of the listed items. In some embodiments, for any of the foregoing methods, the method further comprises comparing the level of at least one of the one or more amyloid-producing bacterial species in the subject to a reference level of at least one of the one or more amyloid-producing bacterial species in a non-PD subject (or two or more non-PD subjects). In some embodiments according to the methods and compositions described herein, the one or more non-PD subject are healthy adults. In some embodiments according to the methods and compositions described herein, the amyloid comprises, consists essentially of, or consists of an amyloid subunit of curli (CsgA). In some embodiments according to the methods and compositions described herein, the CsgA comprises, consists essentially of, or consists of the amino acid sequence of any one of SEQ ID NOs: 1-8, or a variant thereof.

In some embodiments, for any of the methods (including diagnostic methods), compositions, and uses described herein, the sample comprises stool, urine, gut biopsy, cerebral spinal fluid (CSF), CNS biopsy, peripheral nervous tissue biopsy, tissue derived from the vagus nerve, urinary or urogenital tissue biopsy, an *in vitro* culture of any of the listed items, or two or more of any of the listed items. In some embodiments, the sample comprises stool, urine, blood, saliva, mucous, gut biopsy, cerebral spinal fluid (CSF), CNS biopsy, peripheral nervous tissue biopsy, urinary or urogenital tissue biopsy, or an *in vitro* culture of any of the listed sample types. In some embodiments, the sample comprises a fresh sample, a fixed sample, or a combination of these. In some embodiments, the sample comprises a stool sample. In some embodiments, the sample comprises tissue derived from the vagus nerve.

In some embodiments according to any of the methods (including diagnostic methods), compositions, and uses described herein, the subject does not have an identified genetic risk of parkinsonism. In some embodiments according to the methods and compositions described herein, the method is an *in vitro* method.

In some embodiments not part of the invention, the present disclosure provides compositions comprising an amyloid inhibitor for use as (or in the manufacture of) a medicament for treating parkinsonism in a subject. In some other embodiments not part of the invention, the present disclosure provides compositions comprising an amyloid inhibitor for use as (or in the manufacture of) a medicament for delaying or reducing the likelihood of onset of parkinsonism in a subject at risk of developing parkinsonism. In some other embodiments not part of the invention, the present disclosure provides compositions comprising an amyloid inhibitor for use as (or in the manufacture of) a medicament for improving a motor deficit in a subject suffering from parkinsonism.

In some embodiments, for any of the foregoing methods, compositions, and uses, the amyloid inhibitor is an inhibitor of a bacterial amyloid protein. In some embodiments, for any of the foregoing methods, the amyloid inhibitor inhibits the interaction of a bacterial amyloid protein or a subunit thereof with synuclein. In some embodiments, for any of the foregoing methods, the amyloid inhibitor inhibits the seeding of synuclein aggregation by a bacterial amyloid protein or a subunit thereof. In some embodiments, for any of the foregoing methods, the amyloid inhibitor protein comprises a curli subunit. In some embodiments, for any of the foregoing methods, the amyloid inhibitor comprises, consists of, or consists essentially of the amino acid sequence of any one of SEQ ID NOs: 1-8, or a variant thereof. In some embodiments, for any of the foregoing methods, the parkinsonism is a primary or idiopathic parkinsonism, secondary or acquired parkinsonism, hereditary parkinsonism, Parkinson's disease, Parkinson's disease plus syndromes or multiple system degeneration, or any combination thereof. In some embodiments, the for any of the foregoing methods, wherein the parkinsonism is Parkinson's disease.

### BRIEF DESCRIPTION OF THE DRAWINGS

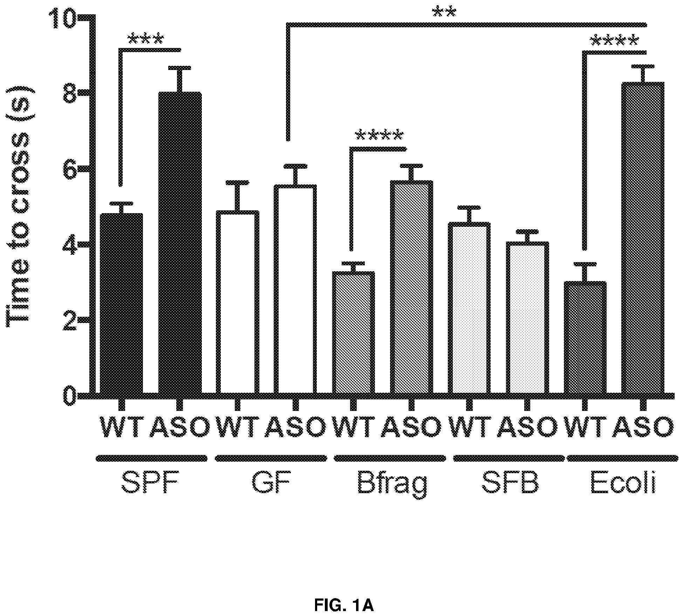

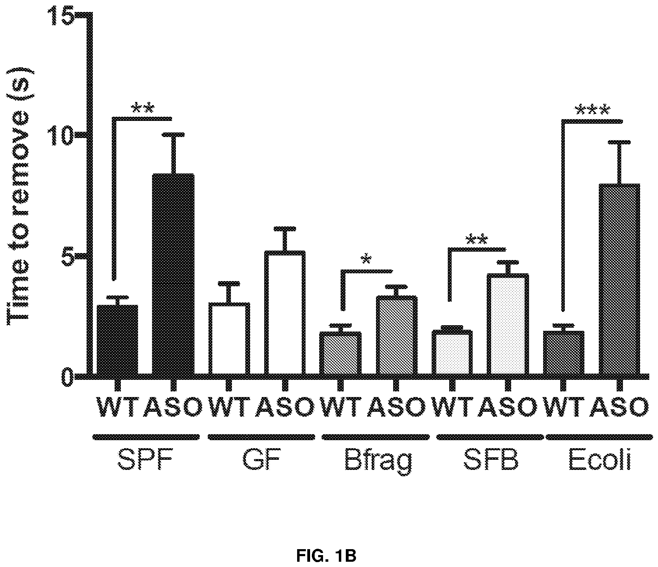

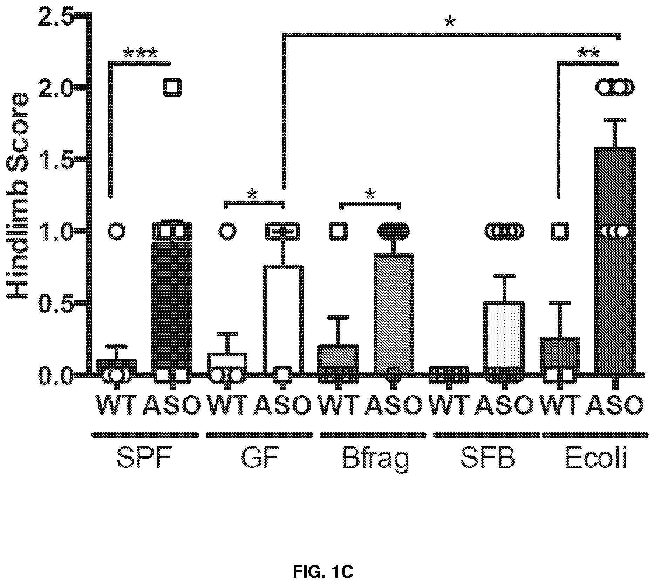

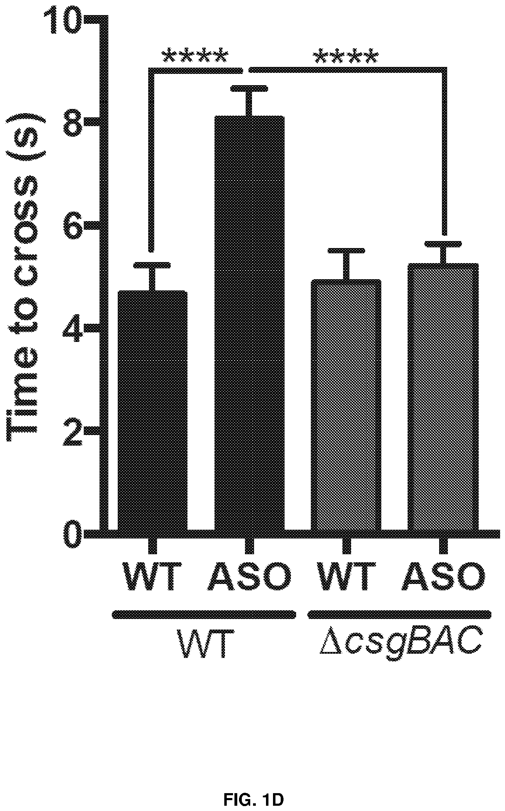

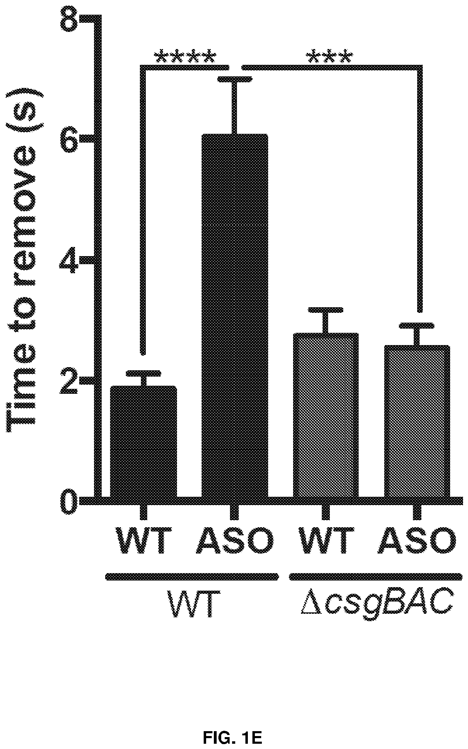

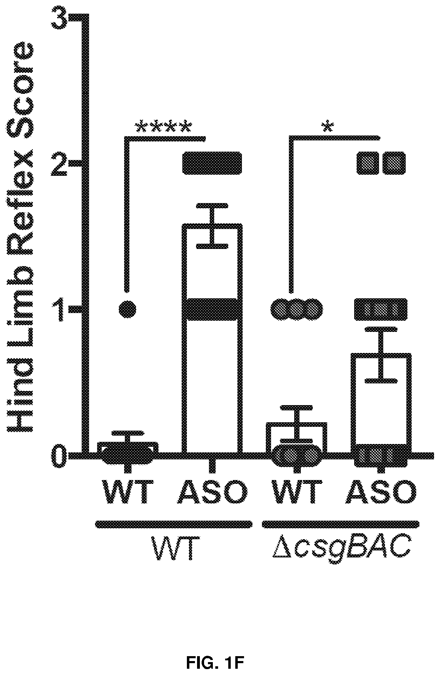

Figures 1A-F show motor function data for wild-type (WT) or α-synuclein overexpressing (ASO) mice following monocolonization. **Fig. 1A** shows performance traversing a balance beam. **Fig. 1B** shows performance removing an adhesive from the nasal bridge. **Fig. 1C** shows hindlimb reflexes. **Fig. 1D** shows performance traversing a balance beam in animals colonized with *E*. *coli* lacking the curli-amyloid (Δ*csgBAC*) compared to animals colonized with wild-type, amyloid producing *E*. *coli* (WT). **Fig. 1E** shows adhesive removal performance in mice colonized with *E. coli* lacking the curli-amyloid (Δ*csgBAC*) compared to animals colonized with wild-type, amyloid producing *E. coli* (WT). **Fig. 1F** shows hindlimb reflexes in mice colonized with *E. coli* lacking the curli-amyloid (Δ*csgBAC*) compared to mice colonized with wild-type, amyloid producing *E*. *coli* (WT).

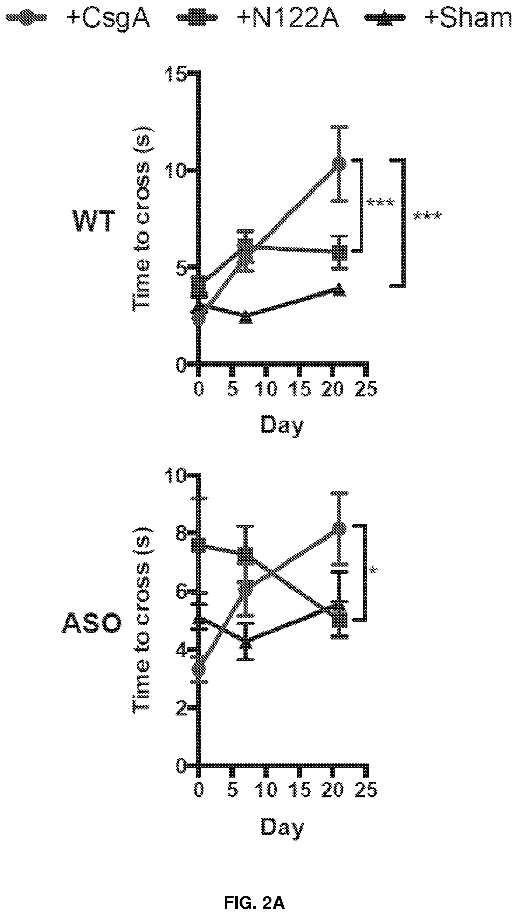

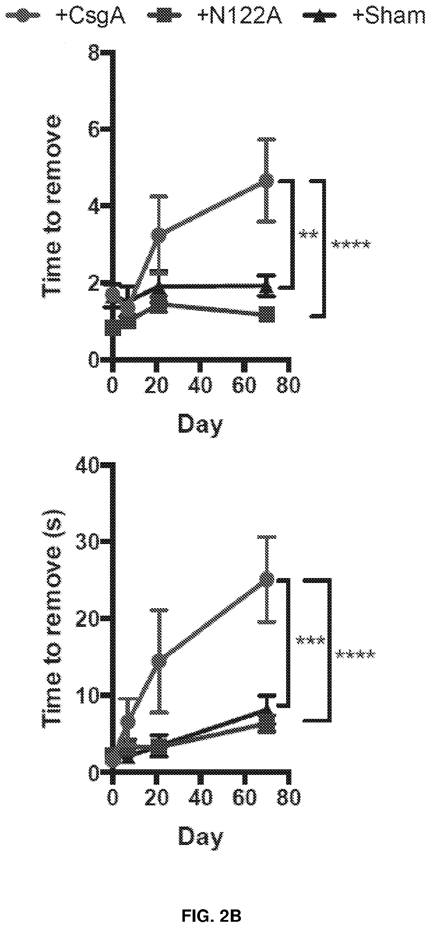

Figures 2A-B show motor function data for wild-type (WT) or α-synuclein overexpressing (ASO) mice following intra-intestinal injection of amyloid-sufficient curli peptide. **Fig. 2A** shows performance in beam crossing. **Fig. 2B** shows performance in an adhesive removal test.

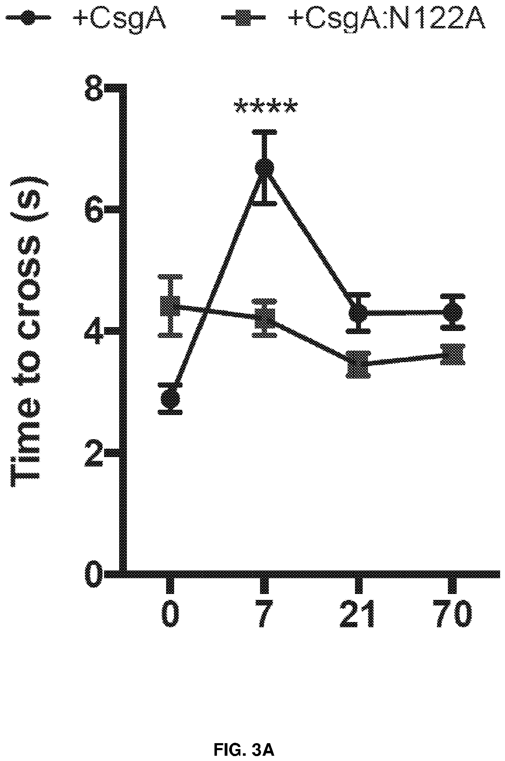

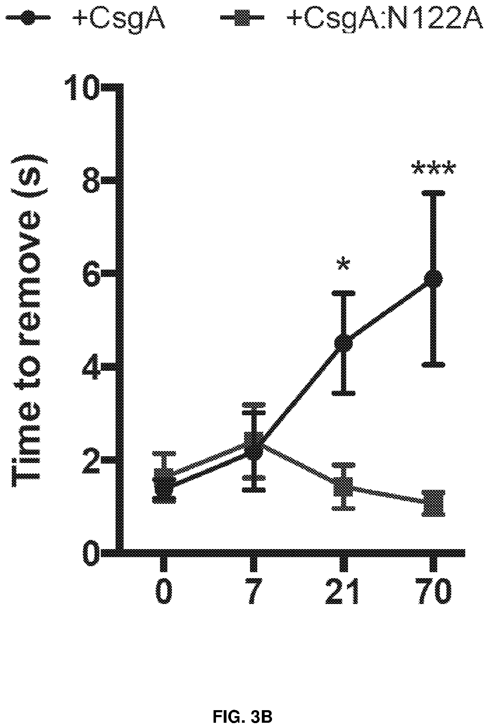

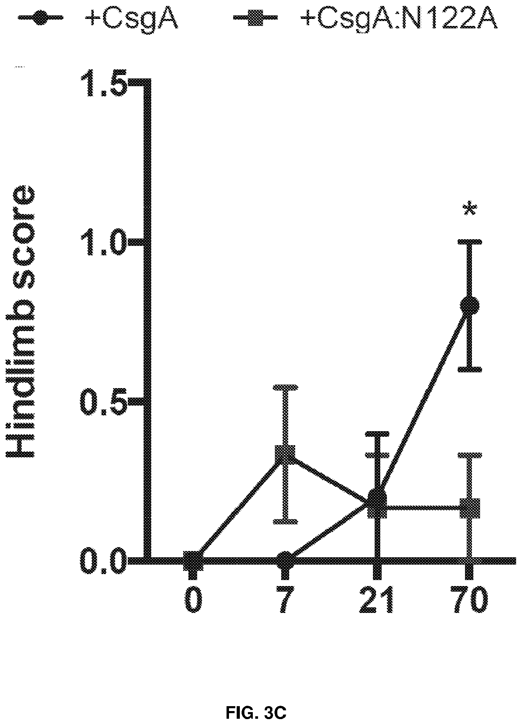

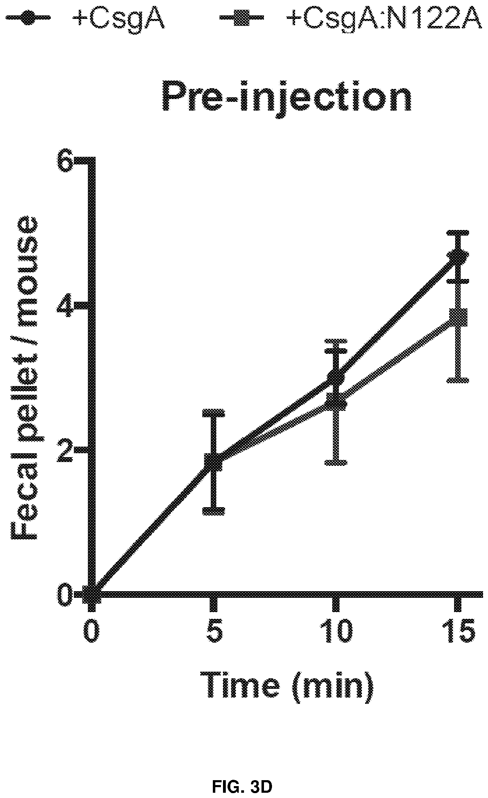

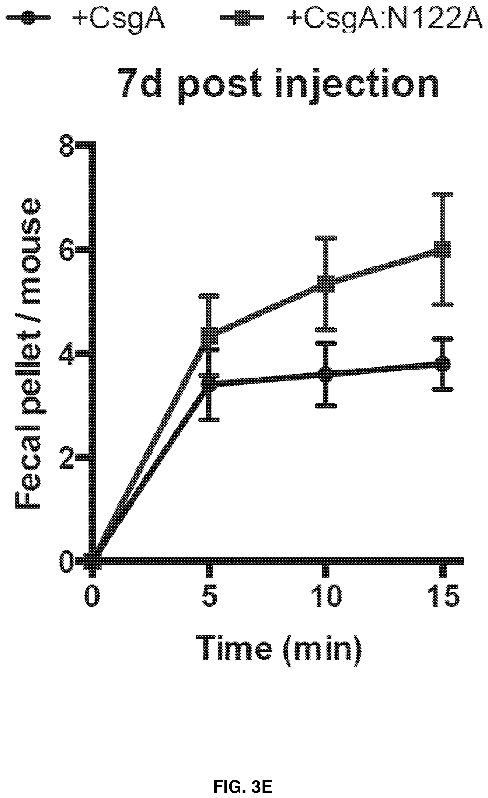

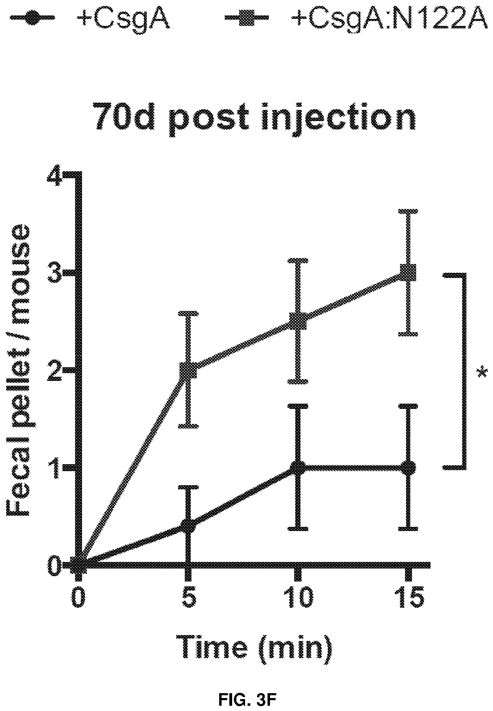

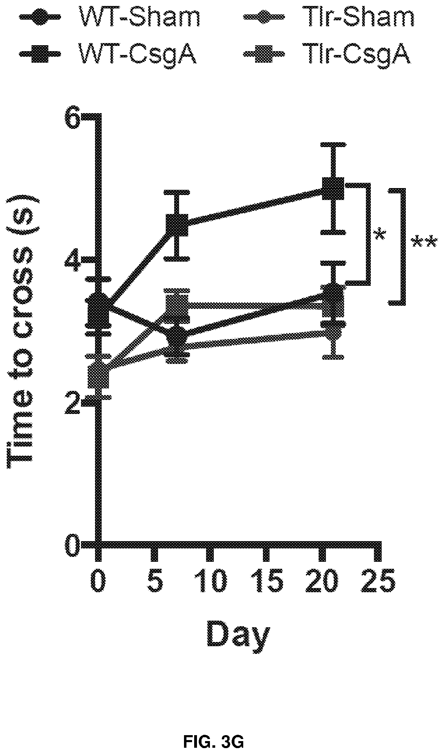

Figures 3A-G show motor function and fecal output in mice upon intra-intestinal injection of either wild-type CsgA. peptide or the N122A mutant. **Fig. 3A** shows dysfunctional beam crossing. **Fig. 3B** shows dysfunctional adhesive removal. **Fig. 3C** shows dysfunctional hindlimb reflexes. **Fig. 3D** shows fecal output pre-injection. **Fig. 3E** shows fecal output 7d post-injection. **Fig. 3F** shows fecal output 70d post-injection. **Fig. 3G** shows that treatment of *Tlr2-*/*-* animals (Tlr) with CsgA peptide does not induce significant motor deficits in the beam crossing assay.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting.

It will be readily understood that the aspects of the present disclosure, as generally described herein, and illustrated in the Figures, can be arranged, substituted, combined, separated, and designed in a wide variety of different configurations, all of which are explicitly contemplated herein.

### Definitions

Unless defined otherwise, technical and scientific terms used herein have the same meaning as commonly understood by one of ordinary skill in the art to which the present disclosure belongs. *See*, *e.g.* Singleton et al., Dictionary of Microbiology and Molecular Biology 2nd ed., J. Wiley & Sons (New York, NY 1994); Sambrook et al., Molecular Cloning, A Laboratory Manual, Cold Springs Harbor Press (Cold Springs Harbor, NY 1989). For purposes of the present disclosure, the following terms are defined below.

As used herein, the term "subject" and variations of this root term, has its customary and ordinary meaning as understood by one of skill in the art in view of this disclosure. It refers to an animal, such as a vertebrate, preferably a mammal. The term "mammal" and variations of this root term, has its customary and ordinary meaning as understood by one of skill in the art in view of this disclosure, and refers to an individual belonging to the class Mammalia and includes, without limitation, humans, domestic and farm animals, and zoo, sports, or pet animals, such as sheep, dogs, horses, cats or cows. In some embodiments, the subject is mouse or rat. In some embodiments, the subject is human. In some embodiments, the subject is a non-human mammal.

As used herein, the term "treatment" and variations of this root term, has its customary and ordinary meaning as understood by one of skill in the art in view of this disclosure. It refers to an intervention (e.g., a clinical intervention) made in response to a disease, disorder or physiological condition manifested by a patient, particularly a patient suffering from a neurodegenerative disease, for example Parkinson's diseases. The aim of treatment may include, but is not limited to, one or more of the alleviation or prevention of symptoms, slowing or stopping the progression or worsening of a disease, disorder, or condition and the remission of the disease, disorder or condition. In some embodiments, "treatment" refers to both therapeutic treatment and prophylactic or preventative measures. Those in need of treatment include those already affected by a disease or disorder or undesired physiological condition as well as those in which the disease or disorder or undesired physiological condition is to be prevented. For example, in some embodiments the treatment may reduce, alleviate, or eradicate the symptom(s) of the disease(s). As used herein, the term "prevention" refers to any activity that reduces the burden of the individual later expressing those parkinsonian symptoms. This can take place at primary, secondary and/or tertiary prevention levels, wherein: a) primary prevention avoids the development of symptoms/disorder/condition; b) secondary prevention activities are aimed at early stages of the condition/disorder/symptom treatment, thereby increasing opportunities for interventions to prevent progression of the condition/disorder/symptom and emergence of symptoms; and c) tertiary prevention reduces the negative impact of an already established condition/disorder/symptom by, for example, restoring function and/or reducing any condition/disorder/symptom or related complications. It is further contemplated that wherever a method of treatment is noted herein, a composition (for example, a composition comprising, consisting of, or consisting essentially of an amyloid inhibitor) for use in the treatment of the noted disease or indication is also expressly contemplated, as is use of the composition for the preparation of a medicament for treating the noted disease or indication.

"Pharmaceutically acceptable" carrier and variations of this root term, has its customary and ordinary meaning as understood by one of skill in the art in view of this disclosure. "Pharmaceutically acceptable" carrier refer to ones which are nontoxic to the cell or mammal being exposed thereto at the dosages and concentrations employed. "Pharmaceutically acceptable" carriers can be, but not limited to, organic or inorganic, solid or liquid excipients which is suitable for the selected mode of application such as oral application or injection, and administered in the form of a conventional pharmaceutical preparation, such as solid such as tablets, granules, powders, capsules, and liquid such as solution, emulsion, suspension and the like. Often the physiologically acceptable carrier is an aqueous pH buffered solution such as phosphate buffer or citrate buffer. The physiologically acceptable carrier may also comprise one or more of the following: antioxidants including ascorbic acid, low molecular weight (less than about 10 residues) polypeptides, proteins, such as serum albumin, gelatin, immunoglobulins; hydrophilic polymers such as polyvinylpyrrolidone, amino acids, carbohydrates including glucose, mannose, or dextrins, chelating agents such as EDTA, sugar alcohols such as mannitol or sorbitol, salt-forming counterions such as sodium, and nonionic surfactants such as Tween^{™}, polyethylene glycol (PEG), and Pluronics^{™}. Auxiliary, stabilizer, emulsifier, lubricant, binder, pH adjustor controller, isotonic agent and other conventional additives may also be added to the carriers.

The pharmaceutically acceptable or appropriate carrier may include other compounds known to be beneficial to an impaired situation of the GI tract, (e.g., antioxidants, such as Vitamin C, Vitamin E, Selenium or Zinc); or a food composition. The food composition can be, but is not limited to, milk, yoghurt, curd, cheese, fermented milks, milk based fermented products, ice-creams, fermented cereal based products, milk based powders, infant formulae, tablets, liquid bacterial suspensions, dried oral supplement, or wet oral supplement.

As used herein, the term "antibody" and variations of this root term, has its customary and ordinary meaning as understood by one of skill in the art in view of this disclosure. It includes human, non-human (e.g., mouse, rat, rabbit, goat, sheep, etc.) and humanized antibodies, and includes polyclonal antibodies, monoclonal antibodies (including full length antibodies which have an immunoglobulin Fc region), antibody compositions with polyepitopic specificity, multispecific antibodies (e.g., bispecific antibodies, diabodies, and single-chain molecules, and antibody fragments (e.g., Fab or F(ab')₂, and F'v) , so long as they exhibit a desired biological activity, for example specific binding to the relevant antigen. In general, an antibody exhibits binding specificity to a specific antigen. For the structure and properties of the different classes of antibodies, see e.g., Basic and Clinical Immunology, 8th Edition, Daniel P. Sties, Abba I. Terr and Tristram G. Parslow (eds), Appleton & Lange, Norwalk, Conn., 1994, page 71 and Chapter 6.

### Neurodegenerative disorders

Neurological dysfunction is the basis of numerous human diseases. Behavioral, psychiatric, and neurodegenerative disorders often display hallmark neuropathologies within the central nervous system (CNS). One neuropathology, amyloidosis, results from aberrant aggregation of specific neuronal proteins that disrupt many cellular functions. Affected tissues often contain insoluble aggregates of proteins that display altered conformations, a feature believed to contribute to an estimated 50 distinct human diseases. Neurodegenerative amyloid disorders, including Alzheimer's, Huntington's, and Parkinson's diseases (PD), are associated with amyloid proteins. PD is the second most common neurodegenerative disease in the United States, affecting an estimated 1 million people and 1% of the US population over 60 years of age. Worldwide, about 3 million patients and caregivers suffer from the often-debilitating symptoms of PD, which involve motor deficits including tremors, muscle rigidity, bradykinesia, and impaired gait. It is a multifactorial disorder that has a strong environmental component, as less than 10% of cases are hereditary. Aggregation of α-synuclein (αSyn) is thought to be pathogenic in a family of diseases termed synucleinopathies, which includes PD, multiple system atrophy, and Lewy body disease. αSyn aggregation is a stepwise process, leading to oligomeric species and intransient fibrils that accumulate within neurons. Dopaminergic neurons of the substantia nigra pars compacta (SNpc) appear particularly vulnerable to effects of αSyn aggregates. Dopamine modulators are a first-line therapeutic in PD; however, treatments can carry serious side effects and often lose effectiveness. Discovery of safe and effective therapeutics are needed to address the increasing burden of PD in an ever-aging population.

Peripheral influences have been implicated in the onset and/or progression of diseases that impact the brain (Dinan and Cryan, 2015). Bidirectional communication between the gut and the brain in anxiety, depression, nociception, and autism spectrum disorder (ASD) has been suggested (Mayer et al., 2014; Schroeder and Backhed, 2016; Sharon et al., 2016). Gastrointestinal (GI) physiology and motility are influenced by signals arising both locally within the gut and from the CNS. Neurotransmitters, immune signaling, hormones, and neuropeptides produced within the gut may, in turn, impact the brain (Selkrig et al., 2014; Wall et al., 2014).

The human body is permanently colonized by microbes on virtually all environmentally exposed surfaces, the majority of which reside within the GI tract. The microbiota can have profound impact on neurodevelopment and the CNS. Germ-free (GF) mice and antibiotic-treated specific- pathogen-free (SPF) mice are altered in hippocampal neurogenesis, resulting in impaired spatial and object recognition. The microbiota regulates expression of the 5-hydroxytryptamine receptor (5-HT1A), brain-derived neurotropic factor (BDNF), and NMDA receptor subunit 2 (NR2A). GF mice have altered cortical myelination and impaired blood-brain barrier function. Additionally, the microbiota promotes enteric and circulating serotonin production in mice and affects anxiety, hyperactivity, and cognition. Fecal and mucosa-associated gut microbes are different between individuals with PD and healthy controls.

Gut bacteria can control the differentiation and function of immune cells in the intestine, periphery, and brain. Subjects with PD exhibit intestinal inflammation, and GI abnormalities such as constipation often precede motor defects by many years. Break's hypothesis posits that aberrant αSyn accumulation initiates in the gut and propagates via the vagus nerve to the brain in a prion-like fashion. This notion is supported by pathophysiologic evidence: αSyn inclusions appear early in the enteric nervous system (ENS) and the glossopharyngeal and vagal nerves, and vagotomized individuals are at reduced risk for PD. Further, injection of αSyn fibrils into the gut tissue of healthy rodents is sufficient to induce pathology within the vagus nerve and brainstem.

### Treatment of Neurodegenerative Disorders

The complex population of indigenous gut bacteria produces myriad metabolites and proteins, including functional amyloid proteins. One such bacterial amyloid, curli, is a component of the extracellular matrix and plays a role in attachment to host and abiotic surfaces. While the importance of bacterial amyloids in gastrointestinal (GI) disease is established, whether these bacterially-produced amyloid proteins directly interact with host amyloids and alter the initiation or progression of mammalian amyloid diseases is unknown. Data disclosed herein demonstrate in a model of the human amyloid disease, Parkinson's disease (PD) that animals intestinally-colonized with curli producing bacteria develop hallmark motor dysfunction and pathology. As disclosed herein, the amyloid subunit of curli (CsgA) is capable of directly seeding or nucleating αSyn aggregation, driving the molecular pathology of disease. Examples of CsgA peptides suitable for methods (including diagnostic methods) and uses in accordance with embodiments herein include art-recognized CsgA peptides, for examples polypeptides comprising, consisting essentially of, or consisting any of SEQ ID NOs: 1-8, or a functional fragment or a variant thereof. As used herein, a "variant" of any of SEQ ID NOs: 1-8 refers to a polypeptide comprising a deletion, truncation, and/or one or more point mutations, for example, 1, 2, 3, 4, 5, 6, 7, 8, 9, 10, 11, 12, 13, 14, or 15 point mutations, including ranges between any two of the listed values (e.g., 1-2, 1-3, 1-5, 1-7, 1-10, 2-3, 2-5, 2-7, 2-10, 3-5, 3-7, 3-10, 5-7, 5-10, or 7-10 point mutations in any one of SEQ ID NOs: 1-8), so that the variant polypeptide has at least 85% identity to at least one of SEQ ID NOs: 1-8. In some embodiments, the variant polypeptide has at least 85% identity to any one of SEQ ID NOs: 1-8, for example at least about 85%, 86%, 87%, 88%, 89%, 90%, 91%, 92%, 93%, 94%, 95%, 96%, 97%, 98%, or 99% identity, including ranges between any two of the listed values. In some embodiments, one or more of the point mutations represent conservative mutations. A single intestinal administration of an amyloidogenic CsgA peptide, but not an amyloid-deficient peptide, results in progressive, long-lasting motor dysfunction and pathology in wild-type animals, suggesting that indeed amyloidogenic bacterial proteins can drive synuclein-mediated disease (*See* Examples 2 and 3). Since genetic determinants for PD explain less than 10% of cases, and external environmental factors for αSyn aggregation and PD pathogenesis are largely thought to be responsible, these findings indicate that the production of functional bacterial amyloids in the mammalian GI tract is a previously unrecognized risk factor for PD.

As described herein, bacterial derived amyloids from the gut are capable of cross-seeding synuclein aggregation and potentiating motor dysfunction during Parkinson's disease. Generation of inhibitors to prevent the interaction between bacterial amyloids and synuclein, and seeding of αSyn aggregation, can provide therapeutic benefits to subjects suffering from, or at a risk of developing PD.

Disclosed herein but not part of the invention include methods of treating parkinsonism in a subject. The methods, in some embodiments, comprise administering a composition comprising an amyloid inhibitor to a subject suffering from parkinsonism. Methods of delaying or reducing the likelihood of onset of parkinsonism in a subject are also disclosed. The methods, in some embodiments, comprises administering a composition comprising an amyloid inhibitor to a subject at a risk of developing parkinsonism. Also disclosed but not part of the invention are methods for improving a motor deficit in a subject suffering from parkinsonism. The methods, in some embodiments, comprise administering a composition comprising an amyloid inhibitor to a subject at a risk of developing parkinsonism.

The parkinsonism can be, for example, a primary or idiopathic parkinsonism, secondary or acquired parkinsonism, hereditary parkinsonism, Parkinson plus syndromes or multiple system degeneration, or any combination thereof. In some embodiments, the parkinsonism is Parkinson's disease.

The amyloid inhibitor can be, for example, an inhibitor for a bacterial amyloid protein. In some embodiments, the bacterial amyloid comprises a curli subunit. In some embodiments, the amyloid inhibitor inhibits the interaction of a bacterial amyloid protein or a subunit thereof with synuclein. In some embodiments, the amyloid inhibitor inhibits the seeding or nucleation of synuclein aggregation by a bacterial amyloid protein or a subunit thereof.

Also disclosed herein are compositions comprising one or more amyloid inhibitors. The compositions can be, for example, pharmaceutical compositions comprising one or more pharmaceutically acceptable carriers.

### Diagnosis of Parkinsonism

As disclosed herein, identification of amyloid-producing bacteria in subjects (e.g., the gut of the subjects) can be used to diagnose parkinsonism, for example before the onset of severe motor symptoms and/or as a marker for the severity of disease progress, and inform disease prognosis.

Methods of diagnosing parkinsonism in a subject are provided in accordance with some embodiments herein. The methods of diagnosis may also be referred to herein as "diagnostic methods." Furthermore, where diagnostic methods are described herein, diagnostic regents as described herein for *in vitro* use in diagnosis are also expressly contemplated. In some embodiments, the methods comprise determining the presence and/or level of one or more amyloid-producing bacterial species in the subject, whereby the presence and/or abnormal level of the one or more amyloid-producing bacterial species indicates that the subject has a risk of developing, or is suffering from a symptom of parkinsonism. In some embodiments, the parkinsonism is Parkinson's disease. The detection of the presence and/or level of the amyloid-producing bacterial species can be performed by an analytical method as described herein. The amyloid-producing bacterial species can be, for example, an amyloid-producing E. coli. The methods can, in some embodiments, further comprise comparing the level of at least one of the one or more amyloid-producing bacterial species in the subject to a reference level of the at least one of the one or more amyloid-producing bacterial species in one or more non-PD subjects. In some embodiments, the non-PD subjects are healthy adults. It is contemplated that in methods and uses of some embodiments herein, the presence or level of amyloid-producing bacterial species is determined by detecting bacterially-produced amyloid polypeptides (for example, a polypeptide comprising, consisting essentially of, or consisting or any of SEQ ID NOs: 1-8 or a variant or functional fragment thereof), for example in a sample of the subject *in vitro*, or by detecting a unique characteristic of a bacterial strain that produces the amyloids (for example a nucleic acid encoding a CsgA peptide, such as a polypeptide comprising, consisting essentially of, or consisting or any of SEQ ID NOs: 1-8 or a variant or functional fragment thereof). In some embodiments, bacteria from a sample of a subject are cultured, and then the amyloid polypeptides and/or bacterial strain (e.g. a unique characteristics of the bacterial strain) are detected in the culture. In some embodiments, the subject is not known to have parkinsonism. In some embodiments, the subject is not known to have any genetic risk factors of parkinsonism. In some embodiments, a presence of CsgA peptide, a nucleic acid encoding a CsgA polypeptide, or an amyloid-producing bacterial strain in direct culture, is indicative of parkinsonism in the subject. In some embodiments, a level of CsgA peptide, a nucleic acid encoding a CsgA polypeptide, or amyloid-producing bacterial strain in direct culture that is greater than the level in a comparable sample of a control individual known not to have parkinsonism (e.g., a sample from the same tissue type or types in the control individual) is indicative of parkinsonism in the subject.

In diagnostic methods and uses in accordance with some embodiments, the presence and/or level of one or more amyloid-producing bacterial species is determined in a subject. It is noted that determining the presence/and or level of amyloid-producing bacterial species in a subject in accordance with diagnostic methods and uses of various embodiments herein can be performed indirectly, for example by analyzing a sample of the subject *in vitro*, and as such, the method is not necessarily an *in vivo* method. In some embodiments, said determining the presence and/or level of one or more amyloid-producing bacterial species is carried out *in vitro*, for example in a sample as described herein, according to analytical methods disclosed herein and/or according to those methods that are known and/or routinely practiced in the art. In some embodiments the presence and/or abnormal level of the one or more amyloid-producing bacterial species can be identified in biological samples derived from a subject. Examples of samples that are suitable for diagnostic methods and uses of some embodiments herein include, but are not limited to, stool samples, urine samples, blood samples, saliva samples, samples of abscess or abscess effluent, samples of spinal, interstitial, or lymphatic fluid, tissue biopsy samples, and combinations of two or more of any of these. Exemplary tissue biopsy samples that are suitable for diagnostic methods and uses of some embodiments herein may comprise one or more of gut biopsy samples, comprising one or more of intestinal endothelial cells or tissue especially of the colon or large intestine; any tissue of the digestive tract including the gallbladder; peritoneal tissue biopsy samples, nerve tissue biopsy samples, especially of enteric nerves or of the vagus nerve, or comprising central nervous system (CNS) tissue; or urinary or renal tissue biopsy samples, including samples of ureter, bladder endothelium, renal loop endothelium, or any tissue of the kidney, bladder, ureter, or urinary/urogenital tract. In some embodiments, the tissue biopsy samples are used for determination of the presence and/or abnormal level of the one or more amyloid-producing bacterial species. In some embodiments, the sample comprises, consists essentially or, or consists of a tissue and/or body that is not from the gut. In some embodiments, the presence/and or level of amyloid-producing bacterial species is detected *in vivo.*

In diagnostic methods and uses in accordance with some embodiments, the presence and/or abnormal level of the one or more amyloid-producing bacterial species can be determined using one or more antibodies specific to said amyloid-producing bacterial species or to proteins, peptides, or metabolites secreted by said species. The skilled artisan will appreciate that such antibodies can be used in accordance with analytical methods as described herein, for example, fluorescence-activated cell sorting (FACS), enzyme-linked immuno-sorbent assay (ELISA), western blot, lateral flow assay, or no-wash assay, or in conjunction with certain mass spectrometric methods. The antibody can be contacted with a sample as described herein in conjunction with an analytical method so as to determine the presence, absence, or level of an amyloid-producing bacterial species, for example by determining a level of the amyloid itself In some embodiments, the presence and/or abnormal level of the one or more amyloid-producing bacterial species can be determined using non-antibody peptides specific to said species or to proteins, peptides, or metabolites produced and/or secreted by said species. In some embodiments, said one or more antibodies, or said non-antibody peptide comprises, consists essentially of, or consists of an antibody or non-antibody peptide that binds specifically to CsgA, CsgB, or CsgG as described herein. In some embodiments, said antibody is an anti-CsgA antibody. In some embodiments, the anti-CsgA antibody binds specifically to any one of SEQ ID NOs: 1-8, and thus specifically binds to a polypeptide comprising, consisting essentially of, or consisting any one of SEQ ID NOs: 1-8. In some embodiments, the anti-CsgA antibody binds specifically to two or more of SEQ ID NOs: 1-8, for example, two, three, four, five, six, or seven of these. In some embodiments, said non-antibody peptide is CsgA, CsgB, or CsgG. In some embodiments, said one or more antibodies and/or said one or more non-antibody peptides may be fused, conjugated, or combined using such methods as are known and/or routinely practiced in the art. In some embodiments, said one or more antibodies and/or said one or more non-antibody peptides may be conjugated to, fused to, or may optionally comprise within their structures, one or more indicator moieties. Exemplary indicator moieties include but are not limited to radiolabels such as ¹⁴C, or ³H; spin labels such as ¹³C or ¹⁵N; fluorescent labels such as fluorescein and its derivatives, quantum dots, or fluorescent proteins (including the Green Fluorescent Protein, GFP and the Red Fluorescent Protein, DsRed); enzymes, such as beta galactosidase, horseradish peroxidase, or luciferase, or colorimetric labels, such as latex beads, quantum dots, or nanoparticles. In some embodiments, said one or more antibodies and/or said one or more non-antibody peptides may be utilized in a dot-blot, slot-blot, western-blot, or lateral flow assay.

In diagnostic methods and uses in accordance with some embodiments, the presence and/or abnormal level of the one or more amyloid-producing bacterial species can be determined by culturing said species. In some embodiments the presence and/or abnormal level of the one or more amyloid-producing bacterial species can be determined using genetic and/or genomic methods, including Polymerase Chain Reaction (PCR)-based methods, including but not limited to quantitative PCR (qPCR), reverse-transcription PCR (RT-PCR) are modifications or variants thereof such as are known and/or routinely practiced in the art. In some embodiments the presence and/or abnormal level of the one or more amyloid-producing bacterial species can be determined by sequencing-based methods such as, for example, sequencing of the 16S ribosomal RNA; by sequencing of other loci as are known in the art to be diagnostic for or unique to curli-producing bacteria; by sequencing of the CsgA gene or Csg operon; or by whole genome sequencing. Said sequencing may be used to infer the presence of curli- producing bacteria within a sample from a subject through the identification of the presence of 16S rRNA genes, CsgA genes, Csg operon sequences, or other sequences as are known in the art to be unique to curli-producing bacteria. Such curli-producing bacteria as are known in the art include but are not limited to *E. coli*, especially strains K-12, H8824, E06/486, 080/01, and 340/97, as well as *Enterobacter cloacae* and *Stigmatella aurcintiaca.* Said sequencing may be carried out utilizing chain termination methods, nanopore methods, or other such sequencing techniques as are known and/or routinely practiced in the art. As understood by one of ordinary skill in the art, said sequencing methods may comprise long-read or short-read "shotgun" sequencing methods as well. Exemplary CsgA genes are those which encode peptides having the sequences provided in any of SEQ ID NOs: 1-8. In some embodiments, a CsgA gene encodes a variant or functional fragment of any of SEQ ID NOs: 1-8.

In diagnostic methods and uses in accordance with some embodiments the presence and/or abnormal level of the one or more amyloid-producing bacterial species can be determined by additional analytical methods as are known and/or routinely practiced in the art. Exemplary analytical methods suitable for the determination of the presence and/or abnormal level of the one or more amyloid-producing bacterial species (which may be referred to herein as "analytical methods") include, but are not limited to, direct culture or co-culture of bacteria, PCR (for example, quantitative or qualitative PCR), fluorescence in-situ hybridization (FISH), microarray analysis, fluorescence-activated cell sorting (FACS), a mass spectrometric methods such as matrix-assisted laser desorption-ionization/time-of-flight (MALDI-TOF) or a related method, enzyme-linked immuno-sorbent assay (ELISA), western blot, lateral flow assay, and no-wash assay. In some embodiments, the analytical method comprises PCR (for example, quantitative or qualitative PCR), fluorescence in-situ hybridization (FISH), or microarray analysis. In some embodiments, the analytical method comprises fluorescence-activated cell sorting (FACS), a mass spectrometric method such as matrix-assisted laser desorption-ionization/time-of-flight (MALDI-TOF) or a related method, enzyme-linked immuno-sorbent assay (ELISA), western blot, lateral flow assay, or no-wash assay. In some embodiments, the analytical method comprises direct culture.

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. The invention is defined by the appended claims.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e*.*g*., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g*., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g*., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g*., " a system having at least one of A, B, or C" would include but not be limited to systems that have A alone, B alone, C alone, A and B together, a and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a nonlimiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

### Examples

**Example 1.** As shown in Figures 1A-F, wild-type (WT) or α-synuclein overexpressing (ASO) mice were monocolonized with Bacteroides fragilis (Bfrag), segmented filamentous bacteria (SFB) or Escherichia coli (E. coli). Animals were tasked with (Figure 1A) traversing a balance beam, (Figure 1B) removing an adhesive from the nasal bridge, or (Figure 1C) hindlimb reflexes were measured. Only ASO animals colonized with a complex microbiota (SPF) or with E. coli displayed significant motor deficits. Similarly, ASO animals colonized with E. coli lacking the curli-amyloid (ΔcsgBAC) do not display significant motor deficits in the (Figure 1D) beam, (Figure 1E) adhesive removal, or (Figure 1F) hindlimb reflexes compared to animals colonized with wild-type, amyloid producing E. coli (WT). This data shows that monocolonization with amyloid-producing E. coli is sufficient to promote synuclein-mediated motor dysfunction.

**Example 2.** As shown in Figures 2A-B, wild-type (WT) or α-synuclein overexpressing (ASO) mice were intra-intestinally injected with wild-type, amyloid-sufficient (e.g., amyloid-forming) CsgA peptide (+CsgA) and motor function was measured over time in the (Figure 2A) beam crossing and (Figure 2B) adhesive removal test. ASO mice injected with CsgA displayed worsened motor function compared to non-injected (Sham). Additionally, injection of a mutant CsgA peptide, which cannot form amyloid (N122A), did not induce motor deficits. Surprisingly, WT animals, not predisposed to motor dysfunction, also displayed a decrease in motor function when treated with CsgA, but not the N122A mutant. This data shows that intra-intestinal injection of amyloid-sufficient curli peptide promotes worsened motor dysfunction.

**Example 3.** As shown in Figures 3A-G, wild-type B6 mice were intra-intestinally injected with either wild-type CsgA peptide or the N122A mutant, and motor function was measured over time. Mice treated with CsgA, but not the mutant, displayed significant motor dysfunction in the (Figure 3A) beam crossing, (Figure 3B) adhesive removal, and (Figure 3C) hindlimb reflexes. Fecal output also began to decline over time (Figures 3D-F). Further, treatment of Tlr2-/- mice (Tlr) with CsgA peptide did not induce significant motor deficits in the beam crossing assay (G). This data shows that CsgA is sufficient to induce motor dysfunction in wild-type animals.

While various aspects and embodiments have been disclosed herein, other aspects and embodiments will be apparent to those skilled in the art.

## Claims

1. An in vitro method of determining if a subject has a risk of developing parkinsonism, comprising
receiving at least one sample derived from a subject, wherein said sample comprises a stool sample;
detecting a bacterial CsgA in the sample; and
determining that the subject has a risk of developing parkinsonism when the detected bacterial CsgA in the sample is at a level higher than the level of bacterial CsgA in a reference sample of a healthy or non-parkinsonism individual.

2. The method of claim 1, wherein the parkinsonism is a primary or idiopathic parkinsonism, secondary or acquired parkinsonism, hereditary parkinsonism, Parkinson's disease, Parkinson's disease plus syndromes or multiple system degeneration, or any combination thereof.

3. The method of claim 1 or 2, wherein the bacterial CsgA is *E. coli* CsgA.

4. The method of any one of claims 1-3, wherein the bacterial CsgA is detected by direct culture, antibody binding, ELISA, western blot, lateral flow assay, no-wash assay, sequencing, PCR, RT-PCR, qPCR or any two or more of these.

5. The method of any one of claims 1-4, wherein detecting the bacterial CsgA comprises detecting a presence and/or level of a nucleic acid encoding the bacterial CsgA in the sample.

6. The method of any one of claims 1-5, wherein detecting the bacterial CsgA comprises detecting a presence and/or level of an aggregate of bacterial amyloid comprising the bacterial CsgA.

7. The method of any one of claims 1-10, wherein the bacterial CsgA comprises an amino acid sequence of any one of SEQ ID NOs: 1-8.

8. The method of any one of claims 1-11, wherein the subject does not have an identified genetic risk of parkinsonism.

## Patentansprüche

1. In-vitro-Verfahren, um zu bestimmen, ob ein Subjekt ein Risiko aufweist, Morbus Parkinson zu entwickeln, Folgendes umfassend:
Empfangen mindestens einer Probe, die von einem Subjekt stammt, wobei die Probe eine Stuhlprobe umfasst;
Detektieren eines bakteriellen CsgA's in der Probe; und
Bestimmen, dass das Subjekt ein Risiko aufweist, Morbus Parkinson zu entwickeln, wenn das detektierte bakterielle CsgA in der Probe auf einem höheren Pegel ist als der Pegel bakteriellen CsgA's in einer Referenzprobe eines gesunden oder nichtparkinsonerkrankten Individuums.

2. Verfahren nach Anspruch 1, wobei der Morbus Parkinson ein primärer oder idiopathischer Morbus Parkinson, ein sekundärer oder erworbener Morbus Parkinson, erblicher Morbus Parkinson, Parkinson'sche Krankheit, Parkinson'sche Krankheit mit Syndromen oder mit Multisystemdegeneration oder eine Kombination davon ist.

3. Verfahren nach Anspruch 1 oder 2, wobei das bakterielle CsgA CsgA von *E. coli* ist.

4. Verfahren nach einem der Ansprüche 1-3, wobei das bakterielle CsgA durch Direktkultur, Antikörperbindung, ELISA, Western Blot, Lateral Flow Assay, No-Wash Assay, Sequenzierung, PCR, RT-PCR, qPCR oder eines oder mehrere dieser detektiert wird.

5. Verfahren nach einem der Ansprüche 1-4, wobei das Detektieren des bakteriellen CsgA's Detektieren eines Vorliegens und/oder Pegels einer Nukleinsäure, die das bakterielle CsgA codiert, in der Probe umfasst.

6. Verfahren nach einem der Ansprüche 1-5, wobei das Detektieren des bakteriellen CsgA's Detektieren eines Vorliegens und/oder Pegels eines Aggregats bakteriellen Amyloids umfasst, das das bakterielle CsgA umfasst.

7. Verfahren nach einem der Ansprüche 1-10, wobei das bakterielle CsgA eine Aminosäurensequenz eines von SEQ ID NOs: 1-8 umfasst.

8. Verfahren nach einem der Ansprüche 1-11, wobei das Subjekt kein identifiziertes genetisches Risiko für Morbus Parkinson aufweist.

## Revendications

1. Procédé in vitro visant à déterminer si un sujet présente un risque de développer un parkinsonisme, comprenant
la réception d'au moins un échantillon provenant d'un sujet, dans lequel ledit échantillon comprend un échantillon de selles ;
la détection d'une CsgA bactérienne dans l'échantillon ; et
la détermination que le sujet présente un risque de développer un parkinsonisme lorsque la CsgA bactérienne détectée dans l'échantillon est à un niveau supérieur au niveau de CsgA bactérienne dans un échantillon de référence d'un individu sain ou non parkinsonien.

2. Procédé selon la revendication 1, dans lequel le parkinsonisme est un parkinsonisme primaire ou idiopathique, un parkinsonisme secondaire ou acquis, un parkinsonisme héréditaire, la maladie de Parkinson, la maladie de Parkinson plus des syndromes ou une dégénérescence systémique multiple, ou toute combinaison de ceux-ci.

3. Procédé selon la revendication 1 ou 2, dans lequel la CsgA bactérienne est une CsgA de *E. coli.*

4. Procédé selon l'une quelconque des revendications 1 à 3, dans lequel la CsgA bactérienne est détectée par culture directe, liaison d'anticorps, ELISA, Western blot, dosage à flux latéral, dosage sans lavage, séquençage, PCR, RT-PCR, qPCR ou deux quelconques d'entre eux ou plus.

5. Procédé selon l'une quelconque des revendications 1 à 4, dans lequel la détection de la CsgA bactérienne comprend la détection d'une présence et/ou du niveau d'un acide nucléique codant pour la CsgA bactérienne dans l'échantillon.

6. Procédé selon l'une quelconque des revendications 1 à 5, dans lequel la détection de la CsgA bactérienne comprend la détection d'une présence et/ou du niveau d'un agrégat d'amyloïde bactérien comprenant la CsgA bactérienne.

7. Procédé selon l'une quelconque des revendications 1 à 10, dans lequel la CsgA bactérienne comprend une séquence d'acides aminés de l'une quelconque des SEQ ID N° : 1 à 8.

8. Procédé selon l'une quelconque des revendications 1 à 11, dans lequel le sujet ne présente pas de risque génétique identifié de parkinsonisme.
